# EUROPEAN PATENT APPLICATION

(11) **EP 3 549 597 A1**
(43) Date of publication of application: **09.10.2019**
(21) Application number: 18166111.7
(22) Date of filing: 06.04.2018
(51) Int. Cl.: A61K 38/17, A61K 39/00, C07K 14/705, C07K 16/00, A61K 47/68, A61P 37/06, A61P 35/00

(54) **SYNTHETIC SIGNALLING CONSTRUCTS AND ITS USE**

(71) Applicant: Heinrich-Heine-Universität Düsseldorf, 40225 Düsseldorf (DE)
(72) Inventor: Scheller, Jürgen, 41469 Neuss (DE); Floss, Doreen, 41468 Neuss (DE); Lang, Philipp, 40591 Düsseldorf (DE); Engelowski, Erika, 40878 Ratingen (DE); Xu, Haifeng, 40225 Düsseldorf (DE); Franke, Manuel, 41460 Neuss (DE); Schneider, Artur, 32547 Bad Oeynhausen (DE)
(74) Representative: Gramm, Lins & Partner Patent- und Rechtsanwälte PartGmbB

(57) **Abstract**

In a first aspect, the present invention relates to new recombinant artificial polypeptides allowing cytoplasmic signaling in cells containing the same. In particular, the present invention relates to a recombinant polypeptide containing a domain with a first binding partner selected from an artificial ligand and a receptor binding an artificial ligand, a transmembrane domain and a cytoplasmic signaling domain whereby the domain with the binding partner and the cytoplasmic signaling domain are a combination of domains not naturally occurring in an organism. In a further aspect, a nucleic acid molecule comprising a nucleic acid sequence encoding the polypeptide according to the present invention as well as a vector, cell, cell line or host cell containing said vector are provided. In addition, the present invention relates to the use of the recombinant polypeptide according to the present invention in treating cancer or autoimmune disease or allowing immune modulation of a subject as well as for use to change the status of cells when applying the specific second binding partner of the first binding partner present in the recombinant polypeptide according to the present invention.

## Description

In a first aspect, the present invention relates to new recombinant artificial polypeptides allowing cytoplasmic signaling in cells containing the same. In particular, the present invention relates to a recombinant polypeptide containing a domain with a first binding partner selected from an artificial ligand and a receptor binding an artificial ligand, a transmembrane domain and a cytoplasmic signaling domain whereby the domain with the binding partner and the cytoplasmic signaling domain are a combination of domains not naturally occurring in an organism. In a further aspect, a nucleic acid molecule comprising a nucleic acid sequence encoding the polypeptide according to the present invention as well as a vector, cell, cell line or host cell containing said vector are provided. In addition, the present invention relates to the use of the recombinant polypeptide according to the present invention in treating cancer or autoimmune disease or allowing immune modulation of a subject as well as for use to change the status of cells when applying the specific second binding partner of the first binding partner present in the recombinant polypeptide according to the present invention.

### Prior Art

Adaptive cell therapy and adaptive cell transfer have shown significant efficacy in the treatment of malignancies and can be curative in patients with various diseases including cancer, autoimmune diseases as well as allowing immune modulation in a subject. For example, cancer is the second most common cause of death in Germany and therapy of cancer as well as therapy of autoimmune diseases or immune modulation in general is the aim of intensive research.

Various methods exist for the treatment of cancer including the adaptive cell therapy beside surgery, irradiation or resection. Utilization of the immune system and manipulating the immune system of the subject to be treated allows to treat cancer as well as to cure autoimmune diseases etc.

In adaptive cell therapy usually patient derived T-cells are engineered ex vivo to express particular receptors or activate particular T-cells. Examples of engineered T-cells useful in adaptive T-cell therapy are engineered T-cells expressing a recombinant T-cell receptor or, alternatively, a chimeric antigen receptor (CAR). Said chimeric antigen receptor is typically composed of an extracellular antigen binding domain derived from an antibody and an intracellular T-cell activation domain derived from the T-cell receptor endodomain. In contrast to the physiological T-cell receptor (TCR) the CAR is composed of one single polypeptide chain that combines antigen binding via the extracellular moiety with a T-cell activation machinery provided by the intracellular signaling moiety.

Other approaches to treat cancer and other diseases is the use of drugs allowing to stimulate T-cells or block particular receptors on T-cells, thus, activating or deactivating said T-cells or increasing the efficacy of said T-cells and the immune response against predetermined targets. For example, medicaments against PD1 as well as the PDL1 (the ligand of PD1) are used successfully in the treatment of tumors. However, one of the main adverse effects of immune therapy so far is an unspecific excessive immune activation and excessive immune response typically induced by a so called cytokine storm with severe side effects, e.g. as described for drugs being anti-CD28 drugs. It was considered that CAR molecules may overcome said problem. The antibody derived domain present in the CAR modified T-cells allows to recognize their target, usually, a cell surface antigen, independently of the Major Histocompatibility Complex (MHC) presentation of antigen and are not compromised by tumor cell variants with lowered or deficient antigen processing which represents a commonly observed mechanism of tumor immune escape. Recently, the first method based on this CAR T-cell tumor immune therapy was approved by the FDA for pediatric acute lymphatic leukemia as well as for the large B-cell lymphoma.

However, CAR based therapy still has problems with respect to specificity and unwanted immune activity including the cytokine storm as described for anti CD19 CAR. Hence, further developments in cell based therapy are required. In particular, it is desired to provide further tools to allow enhanced proliferation of the engineered cells, in particular engineered tumor reactive T-cells while reducing the severe adverse side effects.

A further approach in the art is the use of modified cytokine receptors or modified cytokine induced signal transduction. Cytokine induced signal transduction is executed by natural biological switches among many other functions controlling immune related processes. In principle, cytokine receptors are in an off state in the absence of cytokines and in an on state in the presence of cytokines. The on state might be interrupted by a negative feedback mechanism of depletion of the cytokine and cytokine receptor. For example, ligand independent synthetic receptors based on fusion of leucine zippers or IL-15/sushi and the IL-6 signal transducer gp130 which are locked in the on state have been reported. However, these synthetic or artificial receptors are not switchable, Suthaus, J. et al., Mol Biol Cell, 2010, 21, 2797-2801. Interestingly, a marked activation of IL-6/IL-11 signaling in inflammatory hepatocellular adenomas was directly caused by gain-of-function mutations in the gp130 receptor chain, leading to ligand-independent constitutively active gp130 receptors. Further, switchable synthetic cytokine receptors have been described resulting in gp130-induced signaling by stimulation with the cytokine erythropoietin, Gerhartz, C. et al, J Biol Chem, 1996, 271, 12991-12998.

The major drawback of this system was that erythropoietin has cross-reactivity with its natural EPO-receptors limiting its application both in vitro and in vivo. Also, higher ordered multi-receptor complexes cannot be assembled using natural ligands such as erythropoietin, which only induces receptor-homodimerization. This represents a general problem associated with the use of unmodified naturally occurring ligands, like cytokines. Direct intracellular activation of signal transduction and induction of cell death was achieved using cell permeable, synthetic ligands like FK506 and binding proteins, like FKBP12 resulting in homodimerization and homooligomerization. A problem was, however, that the extent of oligomerization was not controllable. Various formats of synthetic transmembrane receptors have been designed to optimize engineered CAR T-cell responses, including co-stimulatory receptors, notch-based receptors and antigen-specific inhibitory receptors, e.g. Federov, V.D., et al, Sci Tranl Med, 2013, 5, 215ra172, doi:10.1126/scitranslmed.3006597. However, a switchable background-free system, in particular, a free synthetic cytokine receptor system with full control over the assembly modus of the receptor complexes, including hetero/homo-dimeric, -trimeric, or -multimeric organization is not available.

Recently developed nanobodies specifically recognizing GFP and mCherry fail do bind endogenous ligands, see e.g. Fridy P.C., et al, Nat Methods, 2014, 12, 1253-1260, and, thus qualify as binding partners of synthetic cytokine receptors. The N-terminal region of camelidae heavy chain antibodies contains a dedicated variable domain, referred to as VHH or nanobodies, which binds to its cognate antigen. Nanobodies are single domain antibodies of about 110 amino acid residues generated from the variable regions of the heavy chain antibodies.

In view of the above, there is still a need for new approaches in tumor immune therapy as well as for treating other immune-based diseases including autoimmune diseases as well as for immune modulation in a subject in particular, overcoming the adverse side effects described e.g. in CAR expressing T-cells in an adaptive therapy hindering further development of respective therapy. That is, the CAR based adaptive therapy using engineered T-cells expressing the CAR which do not discriminate between malignant cancer cells and healthy cells, a cytokine storm is described for this CAR approaches as well.

### Brief description of the present invention

The present invention relates to a newly developed recombinant polypeptide having reduced toxicity, high specificity, reduced adverse side effect as well as being switchable by simple activation and deactivation.

In a first aspect, the present invention provides a recombinant polypeptide containing at least the following domains starting from the N-terminus to the C-terminus: a first domain containing a first binding partner selected from an artificial ligand and a receptor binding an artificial ligand;
optionally a spacer domain,
a transmembrane domain, and
a cytoplasmic signaling domain wherein the first domain and the cytoplasmic signaling domain is a combination of domains not naturally occurring in an organism.

In a preferred embodiment the recombinant polypeptide according to the present invention comprises a first domain containing a nanobody, and a transmembrane domain and cytoplasmic signaling domain derived from the same cytokine receptor.

In a further aspect, the present invention relates to a nucleic acid molecule comprising a nucleic acid sequence encoding the polypeptide according to the present invention. Further, a vector comprising the nucleic acid sequence according to the present invention, in particular, in form of a viral vector or a plasmid is disclosed.

Moreover, cell, cell lines or host cells containing the vector according to the present invention or the nucleic acid molecule according to the present invention and, eventually, expressing the recombinant polypeptide according to the present invention are described.

Further, the present invention relates to the recombinant polypeptide, the nucleic acid molecule, the vector, the cell, cell line or host cell according to the present invention for use in treating cancer or autoimmune disease or for use in immune modulation of a subject. Further, the above are useful for changing the status of cells including activation and deactivation, inducing apoptosis or cell death in cells as well inducing senescence etc. Further, necrosis like necroptosis may be induced. Moreover, inhibition of cells and exhaustion of cells may be possible.

Finally, the present invention relates to a kit or system containing a vector, a cell, cell line or host cell, a nucleic acid and/or a peptide according to the present invention, in particular for use in the production of cells like T-cells for immune modulation.

### Brief description of the drawings

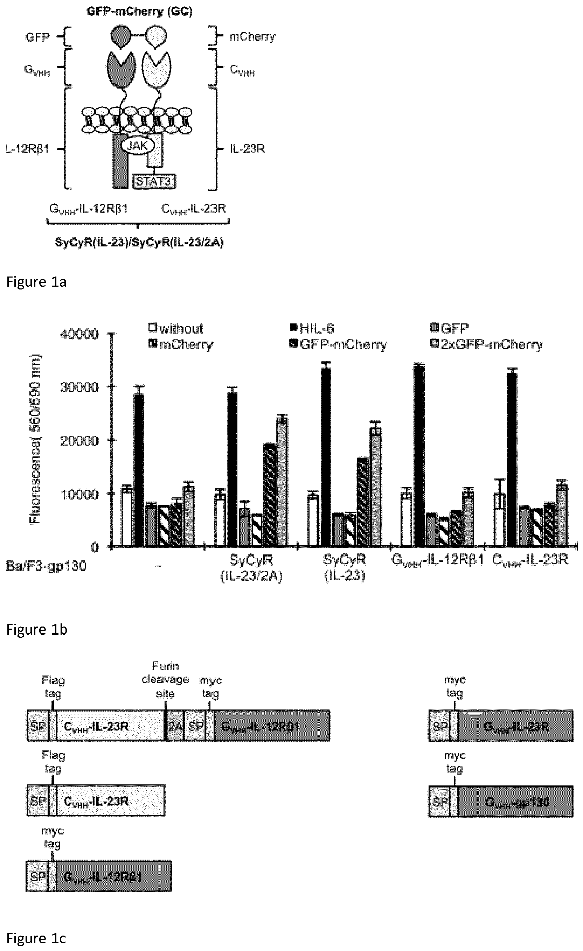

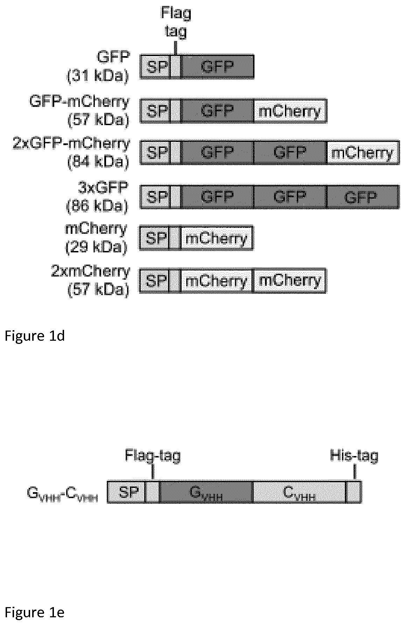

Fig. 1: Synthetic cytokine receptors for IL-23 (SyCyR(IL-23/2A)) simulate IL-23-induced signal transduction and cellular proliferation in transduced Ba/F3-gp130 cells. **a** Schematic illustration of the SyCyR simulating IL-23 signal transduction. The GFP-mCherry fusion protein served as synthetic cytokine ligand. G_{VHH}-IL-12Rβ1 consists of the GFP-nanobody (G_{VHH}) fused to 15 aa of the extracellular part, the transmembrane and intracellular domains of the IL-12Rβ1. C_{VHH}-IL-23R consists of the mCherry-nanobody (C_{VHH}) fused to 17 aa of the extracellular part, the transmembrane and intracellular domains of the IL-23R. The natural STAT3 binding site within the IL-23R was highlighted by the boxed STAT3. **b** Cellular proliferation of different Ba/F3-gp130 cell lines with HIL-6 and GFP:mCherry fusion proteins. Equal numbers of cells were cultured for 3 days in the presence of the indicated synthetic ligands (6.25 ng/ml). Stimulation with mCherry was made with the same volume as with GFP (0.25%). Stimulation with HIL-6 (10 ng/ml) was used as control. Proliferation was measured using the colorimetric CellTiter-Blue Cell Viability Assay. One representative experiment out of three is shown. **c** The expression cassettes for all SyCyRs consist of a signal peptide from IL-11 R followed by a Flag- or myc-tag and the C_{VHH} or G_{VHH}, 13-17 aa of the extracellular part, the transmembrane and intracellular domains of the cytokine receptor. **d** The expression cassettes of the G_{VHH}-C_{VHH} fusion protein consist of a PeIB signal peptide followed by a Flag-tag, G_{VHH}, C_{VHH} and a His tag.

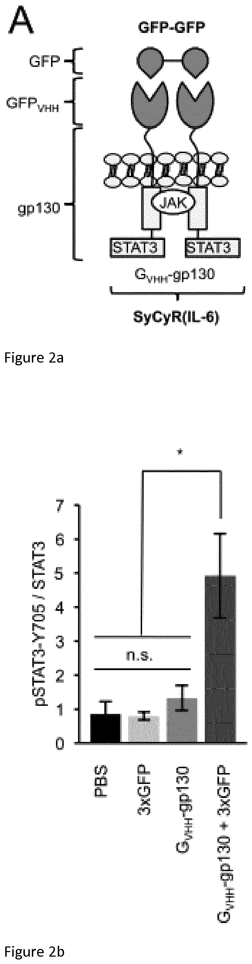

Fig. 2: Synthetic cytokine receptors for IL-6 (SyCyR(IL-6)) simulate IL-6/IL-11-induced signal transduction and cellular proliferation in transduced Ba/F3-gp130 cells and mice. **a** Schematic illustration of the SyCyR simulating IL-6 signal transduction. The GFP-GFP fusion protein served as synthetic cytokine ligand. G_{VHH}-gp130 consists of the GFP-nanobody fused to 13 aa of the extracellular part followed by the transmembrane and intracellular domains of human gp130. The natural STAT3 binding site within gp130 was highlighted by the boxed STAT3. **b** The expression cassettes for all SyCyRs consist of a signal peptide from IL-11R followed by a Flag- or myc-tag and the C_{VHH} or G_{VHH}, 13-17 aa of the extracellular part, the transmembrane and intracellular domains of the cytokine receptor. **c** Ratio of the relative density of pSTAT3 and STAT3 liver expression as determined by immunoblotting shown in F; n = 3 animals/group; *p < 0.05, one-way ANOVA. (H) qRT-PCR of the liver acute phase response Saa1 mRNA 24 h after hydrodynamic transfection of 1.28 µg pcDNA3.1-3xGFP and/or 2.3 µg pcDNA3.1-G_{VHH}-gp130 plasmid DNA in C57BL/6 mice. Data were normalized and calculated using the housekeeper mRNA Gapdh and the ΔΔCT method; n = 6 animals/group; *p < 0.05, one-way ANOVA.

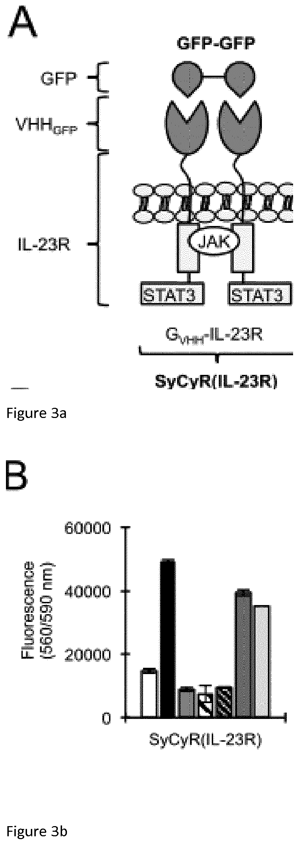

Fig. 3: Synthetic cytokine receptors for homodimeric IL-23R (SyCyR(IL-23R)) induced signal transduction and cellular proliferation in transduced Ba/F3-gp130 cells. **a** Schematic illustration of the SyCyR simulating homodimeric IL-23R signal transduction. The GFP-GFP fusion protein served as synthetic cytokine ligand. G_{VHH}-IL-23R consists of the GFP-nanobody fused to 17 aa of the extracellular part, the transmembrane and intracellular domains of the IL-23R. The natural STAT3 binding site within the IL-23R was highlighted by the boxed STAT3. **b** The expression cassettes for all SyCyRs consist of a signal peptide from IL-11 R followed by a Flag- or myc-tag and the C_{VHH} or G_{VHH}, 13-17 aa of the extracellular part, the transmembrane and intracellular domains of the cytokine receptor. **c** Cellular proliferation of Ba/F3-SyCyR(IL-23R) cells with HIL-6 and GFP:mCherry fusion proteins. Equal numbers of cells were cultured for 3 days in the presence of the indicated synthetic ligands (6.25 ng/ml). Stimulation with mCherry was made with the same volume as with GFP (0.25%). Stimulation with HIL-6 (10 ng/ml) was used as control. Proliferation was measured using the colorimetric CellTiter-Blue Cell Viability Assay. One representative experiment out of three is shown.

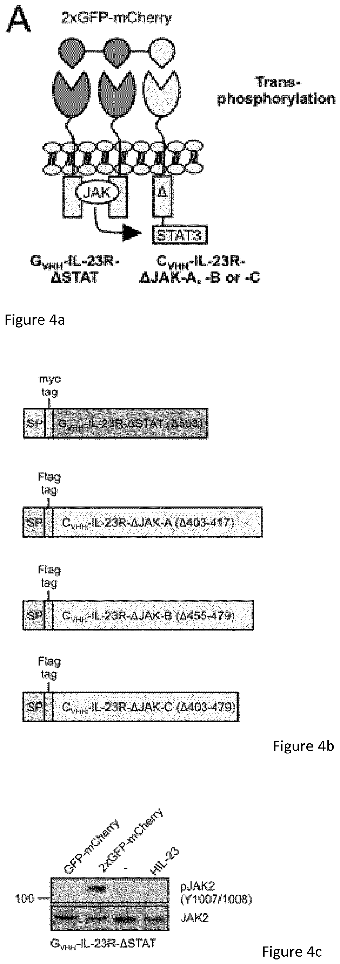

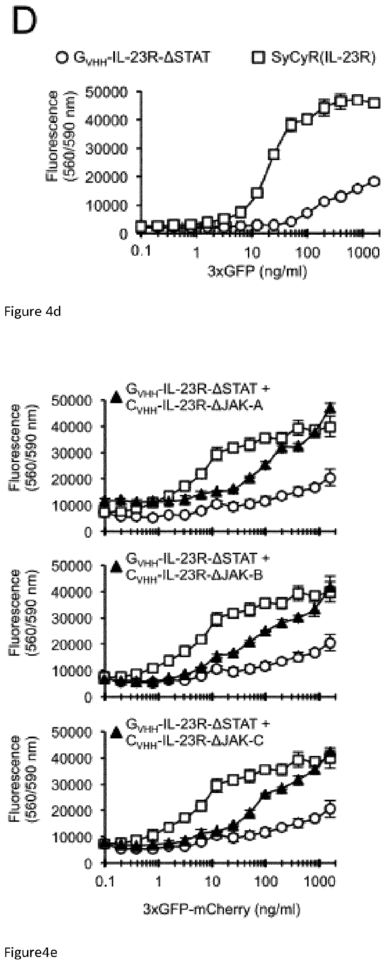

Fig. 4: Engineered heterotrimeric SyCyRs of IL-23R are capable of STAT3 trans-phosphorylation in transduced Ba/F3-gp130 cells. **a** Schematic illustration of IL-23R-SyCyRs simulating STAT3 trans-phosphorylation. The 2xGFP-mCherry fusion protein served as synthetic cytokine ligand. G_{VHH}-IL-23R-ΔSTAT consists of the GFP-nanobody fused to 16 aa of the extracellular part, the transmembrane and intracellular domains of the IL-23R lacking STAT binding motifs. The C_{VHH}-IL-23R-ΔJAK variants consist of the mCherry-nanobody fused to 16 aa of the extracellular part, the transmembrane and intracellular domains of the IL-23R lacking the JAK activation site (ΔJAK-A,-B,-C). **b** The expression cassettes of all trans-activation SyCyRs consist of a signal peptide from IL-11 R followed by a Flag- or myc-tag and the C_{VHH} or G_{VHH}, 16 to 17 aa of the extracellular part, the transmembrane and intracellular domains of the cytokine receptor. **c** Analysis of JAK activation in Ba/F3-IL-23R-ΔSTAT cells. Cells were washed three times, starved, and stimulated with 100 ng/ml of the indicated synthetic ligands for 20 min. Cellular lysates were prepared, and equal amounts of total protein (50 µg/lane) were loaded on SDS gels, followed by immunoblotting using specific antibodies for phospho-JAK2 and JAK2. Western blot data show one representative experiment out of three; **d** a cellular proliferation of Ba/F3-SyCyR(IL-23R), Ba/F3-Gvhh-IL-23delta STAT cells and variants thereof with 3xGFP or 2xGFP-mCherry fusion proteins. Equal numbers of cells were cultured for 3 days in the presence of the indicated ligands (0.1-1600 ng/ml). Proliferation was measured using the colorimetric CellTiter-Blue Cell Viability Assay. One representative experiment out of four is shown.

### Detailed description of the present invention

The present inventors aim in providing new recombinant polypeptides able to induce signal transduction via receptor activation and inactivation in cells containing the same whereby said recombinant polypeptides do not naturally occur in an organism.

In an embodiment of the present invention, nanobodies were used as extracellular domains, namely, as a binding partner for artificial ligands being part of so called synthetic cytokine receptors (SyCyRs), leading to the formation and activation of homo- and heterodimeric and heterotrimeric receptor complexes. The recombinant polypeptides according to the present invention represents switchable receptor systems allowing signaling in target cells.

That is, in a first aspect, a recombinant polypeptide containing at least the following domains starting from the N-terminus to the C-terminus:
a first domain containing a first binding partner selected from an artificial ligand and a receptor binding an artificial ligand;
optionally a spacer domain,
a transmembrane domain, and
a cytoplasmic signaling domain wherein the first domain and the cytoplasmic signaling domain is a combination of domains not naturally occurring in an organism is provided.

As used herein, the term "contain" or "containing" as well as the term "comprise" and "comprising" which are used herein synonymously, include the embodiments of "consist of" and "consisting of".

Unless otherwise indicated, the term "genetically engineered" refers to cells being manipulated by genetic engineering. That is, the cells contain a heterologous sequence which does not naturally occur in said cells or which does not naturally occur in said cells at the specific position introduced in the genome of said cell. Typically, the heterologous sequence is introduced via a vector system or other means for introducing nucleic acid molecules into cells including liposomes. The heterologous nucleic acid molecule may be integrated into the genome of said cells or may be present extrachromosomally, e.g. in the form of plasmids. The term also includes embodiments of introducing genetically engineered, namely, recombinant isolated polypeptides according to the present invention into the cells.

The term "binding pair" as used herein refers to a complex of at least two binding molecules also referred to as binding moieties or binding partner, namely, a first moiety, a first binding partner, of the binding pair and a second moiety, a second binding partner, of the binding pair. Binding moiety and binding partner are used herein interchangeably.

According to the present invention, the first binding partner present in the recombinant peptide according to the present invention binds specifically to the second binding partner, thus, forming a binding pair. The binding pair induces a signal transduction through the cytoplasmic signaling domain present in the recombinant polypeptide according to the present invention.

The recombinant polypeptide is an artificial polypeptide which means that the specific combination of the different domains present in the recombinant polypeptide according to the present invention do not naturally occur in a target organism.

The term "artificial ligand" refers to a ligand which is not naturally present in the environment, in particular, extracellularly, in the organism.

The term "receptor" refers to a binding partner binding specifically to a further binding partner forming specifically a binding pair. Said receptor may be any kind of receptor molecule formed by an amino acid sequence.

As used herein, the term "nanobody" refers to single domain antibodies consisting of a single monomeric variable antibody domain. Typically, the nanobodies are 12 to 15 kilodalton in size. The term "VHH-antibody" or "VHH" are used synonymously with "single domain antibody" or "nanobody".

The term "polypeptide" as used herein refers to a peptide composed of amino acids typically having a size of 500 amino acids at most, like having a size of 400 at most, e.g. 300 at most. For example, the size of the polypeptide is in between 50 and 500 amino acids, like of 100 to 400 amino acids.

The term "derived from" refers to domains, in particular, the cytoplasmic signaling domain, which is obtained or stemming from the particular receptor or molecule mentioned.

The term "fragment thereof" refers to a part of the mentioned peptide having the same desired function, for example, having the same specific binding capacity to a binding partner forming a specific binding pair allowing to transduce signaling through the cytoplasmic signaling domain present in the recombinant polypeptide according to the present invention.

The ligand, namely, the "artificial ligand" refers to a ligand which does not physiologically occur in the environment where the recombinant polypeptide according to the present invention is expressed, typically extracellularly.

In an embodiment, the first binding partner present in the recombinant polypeptide according to the present invention is selected from the group consisting of a single chain antibody unit, a receptor molecule, an endogenous peptide or a fragment thereof and an artificial peptide. Namely, the first binding partner may be a nanobody or other type of single chain antibody unit binding specifically to the epitope of an antigen. Alternatively, two interacting polypeptides, e.g. the binding partner is a receptor molecule having a known ligand, like cytokine receptor molecules.

In another embodiment, the first binding partner is an endogenous peptide or a fragment thereof. For example, the endogenous peptide is a normally intracellularly expressed peptide, like β-catenin, or a fragment thereof. β-catenin is a molecule naturally expressed intracellularly in cells. Hence, extracellular expression of β-catenin or a fragment thereof, namely, a fragment containing an epitope of a β-catenin specific antibody represents an artificial ligand accordingly.

Further, the first binding partner may be an artificial peptide. The person skilled in the art is known of suitable artificial peptides known to form binding pairs with specific peptide or non-peptide binding partners. A typical example of an artificial peptide is avidin or streptavidin with the binding partner biotin.

In a further embodiment, the recombinant polypeptide according to the present invention comprise further a leader sequence being located N-terminally to the first domain containing the first binding partner like a single chain antibody unit.

Generally, the recombinant polypeptide consist of the ectodomain present extracellularly, containing the first domain as described and, optionally, a spacer domain. The ectodomain may be spaced apart from the transmembrane domain by the presence of said spacer domain. Said optional spacer domain links the first domain to the transmembrane domain and it is preferred that said spacer domain in combination with a transmembrane domain is flexible enough to allow the first domain to orient in different directions to facilitate binding pair formation.

The transmembrane domain present in the recombinant polypeptide according to the present invention is typically a hydrophobic alpha helix that spans the membrane. Finally, the endodomain, including the cytoplasmic signaling domain, represents the signaling domain in the cytoplasmic part of the recombinant polypeptide according to the present invention.

The endodomain contains the signaling domain and is also referred to as the intracellular domain which are used herein interchangeably. In an embodiment, the cytoplasmic signaling domain is derived from a cytokine receptor, in particular, is a cytoplasmic signaling domain selected form the group consisting of generally stimulating receptors, e.g. gp130, IL-23R, IL-12Rβ1, IL-7R, IL-13R, IL-15R, IFNαR, IFNγR, TNF1R, TNF2R, EGF-R, IL-22R, silencing (exhausting) receptors, e.g. PD-1, IL-10R, TIM3, IL-27R or killing receptors, e.g. FasR, TRAILR. In a further aspect, the cytoplasmic signaling domain is selected from the group consisting of CD28, CD3ζ chain, CD3ε chain or a signaling domain derived from the B-cell receptor. Further, the domain may contain a costimulatory domain. In an embodiment, the signaling domain is responsible for the activation of the cytotoxic activity in T-cells or interferon gamma secretion by T-cells, respectively, when using the recombinant polypeptide expressed in T-cells, e.g. for use in adaptive cell therapy.

In an embodiment, the transmembrane domain and the cytoplasmic signaling domain is derived from the same molecule, e.g. derived from a cytokine receptor.

In another embodiment, the transmembrane may be obtained from one molecule while the cytoplasmic signaling domain may be obtained from another molecule. E.g. as it is the case in CAR molecules, the transmembrane domain and an intracellular membrane proximal part is derived from CD28 while the intracellular part is derived from CD3ζ. In an embodiment, the CD28 sequence is a mutated sequence as described in the art lacking an LCK binding motif.

The second binding partner binding specifically to the first binding partner present in the recombinant polypeptide according to the present invention is typically an artificial second binding partner. For example, the second binding partner is an artificial ligand in case of the presence of a receptor in the recombinant polypeptide according to the present invention. The term "artificial second binding partner" refers to a molecule not naturally occurring and not being physiologically present in the environment, namely, the extracellular environment of the cell expressing the recombinant polypeptide according to the present invention.

In addition, the present invention provides nucleic acid molecules comprising the nucleic acid sequence encoding the recombinant polypeptide according to the present invention. Furthermore, vectors are provided comprising the nucleic acid sequence according to the present invention encoding the polypeptide as described. The skilled person is well aware of suitable vector systems and vectors, in particular, vectors allowing transfection and transduction of eukaryotic cells, in particular, T-cells. In an embodiment, the vector and plasmids contain two different recombinant polypeptides according to the present invention, thus, allowing to express two different receptor molecules in the genetically engineered cell. By expressing two different recombinant polypeptides it is possible to heterodimerize receptors using suitable artificial ligands. For example, as shown in the examples below, heterodimerization of synthetic cytokine receptors is possible, thus, inducing a signal transduction cascade in said genetically engineered cell and, in addition, allowing switchable signal transduction.

When expressed in a cell, the recombinant polypeptide may represent different types of recombinant peptides as described herein allowing signal transduction, like different types of SyCyRs. For example, stimulating SyCyRs may be expressed allowing activation or proliferation of the respective cells. Further, inhibitory SyCyRs may be expressed inhibiting proliferation or deactivating the cells. Further, the SyCyRs include cell death or apoptosis or senescence inducing signaling domains. Thus, it is possible to actively control the cells and switch the cells from one state to another state, e.g. from an activated or deactivated state and vice versa as well as from a senescence state to a proliferating state and vice versa depending on the presence or absence of specific second binding partners, typically, artificial ligands binding to said SyCyRs.

Examples of stimulating SyCyRs include cytokine signaling domain of the gp130, TNFR, IL-7, inhibitory SyCyRs include cytoplasmic signaling domains derived from IL-27R, PD1 and TIM3R, while cell death inducing SyCyRs includes FASR and TRAIL derived cytoplasmic signaling domains.

**table 1: shown are examples of activation of SyCyR by an artificial ligand accruing to the present tinvention, namely a covalently linked GFP-mCherry Ligand as described in the examples. The respective SyCyR contain nanobody against GFP and mCherry respectively.**

| | | SyCyR-activating ligands | | |
|---|---|---|---|---|
| | Receptor complex | Heterodimeric Ligands type 1+2 | Multimeric Ligands Type 1 | Multimeric Ligands type 2 |
| **STIM-SyCyR** | IL-7R + cγ-chain | + | - | - |
| **INH-SyCyR** | PD-1 | - | + | - |
| **DEATH-SyCyR** | FasR | - | - | + |

For example, the plasmid or vector according to the present invention is a system based on IRES (internal ribosomal entry side). Alternatively, a 2A system may be applied (Fang, J. et al. Nat Biotechnol 23, 584-590, 2005).

The skilled person is well aware of suitable systems allowing transfection and expression of single recombinant polypeptides according to the present invention in a target cell or allowing transfection and expression of two different recombinant polypeptides according to the present invention in one target cell accordingly.

Moreover, the present invention provides a cell, cell line or a host cell containing the vector according to the present invention or a nucleic acid molecule according to the present invention or a nucleic acid molecule according to the present invention or expressing a recombinant polypeptide according to the present invention. Preferably, said cell, cell line or host cell is a T-cell, e.g. a CD4+ T-cell or a CD8+ T-cell. The cell, cell line or host cell according to the present invention is in an embodiment a modified peripheral blood cell like lymphocytes including the mentioned T-cells like CD8+ or CD4+ T-cells. In an embodiment, the cell, cell line or host cell represents a genetically engineered T-cell further expressing a CAR receptor. Using the recombinant polypeptide according to the present invention allows to control the CAR expressing T-cells, thus, improving application of these genetically engineered CAR expressing T-cells in adaptive cell therapy.

In an aspect, the cell, cell line or host cell according to the present invention contains a second vector as defined herein or a second nucleic acid molecule according to the present invention wherein this second vector or nucleic acid molecule encode a polypeptide according to the present invention being different in at least one of the first binding domain or the cytoplasmic signaling domain or both.

In an embodiment, this cell, cell line or host cell according to the present invention may contain a vector or plasmid with two different recombinant polypeptides according to the present invention or containing two vectors or plasmids encoding two different recombinant polypeptides wherein the cytoplasmic signaling domain of the second vector induce signaling through different signaling pathways.

Further, the present invention provides a kit or system containing a vector according to the present invention, the cell, cell line or host cell according to the present invention, the nucleic acid molecule according to the present invention and/or the peptide according to the present invention for use in the production of T-cells expressing the recombinant polypeptide according to the present invention. In particular, said cells are lymphocytes, like T-cells and said cells allow to modulate the immune response in a subject.

Moreover, the present invention provides the recombinant polypeptide according to the present invention, the nucleic acid molecule according to the present invention, the vector according to the present invention or the cell, cell line or host cell according to the present invention for use in treating cancer or autoimmune disease or immune modulation of a subject. For example, the invention allows adaptive cell therapy for treatment of various types of cancer including leukaemia, melanom, lymphom, colon, thyroid, lung, ovary, kidney, breast, neck, stomach, pancreas, esophagus, larynx, pharynx, hepatocellular, prostate, testis, cervix; sarcoma, endometrium, glioblastoma, bile, bladder, mesothelioma, thymus, anaplastic thyroid cancer, basalioma, colorectal, NSC lung cancer, SC lung cancer, merkel cell carcinoma.

Further, the present invention provides recombinant polypeptides according to the present invention, the nucleic acid molecule according to the present invention, the vector according to the present invention or the cell, cell line or host cell according to the present invention for use to change the status of cells in a subject when applying the specific second binding partner of the first binding partner present in said polypeptide or encoded in said vector or by the nucleic acid according to the present invention or expressing the host cell, cell or cell line. In particular the status is a type of a status for use in treating cancer or autoimmune disease or for immune modulation of a subject. In particular, administering the artificial second binding partner allows to change the status of the cells in a switchable manner. This means that an on/off of the signal transduction is possible, thus, switching between e.g. activation and deactivation as well as between senescence and proliferation and, in addition, activation and apoptosis of cell death.

The recombinant polypeptide, the nucleic acid molecule, the vector, the cell, the cell line or host cell according to the present invention is particularly useful in treating cancer or autoimmune disease or for use in immune modulation of a subject in general. That is, the recombinant polypeptide, the nucleic acid molecule, the vector, the cell, cell line or host cell according to the present invention are useful for changing the status of cells. Changing the status of cells includes an activation or deactivation of said cell, inducing any kind of change of the cell like apoptosis or cell death in cells as well as senescence. Further, necrosis, like necroptosis may be induced. Moreover, inhibition of cells including exhaustion of cells, like exhaustion of T cells can be induced. Generally, virotherapy is possible with the recombinant polypeptide, the nucleic acid molecule or the vector according to the present invention. Virotherapy includes the use of virus as therapeutic agents including anti-cancer oncolytic viruses, viral vectors for gene therapy and viral immunotherapy. More generally, virotherapy includes the use of vectors in form of virus to treat medical conditions.

In another embodiment, the polypeptide, the nucleic acid molecule, the vector, the cell or cell line or host cell according to the present invention may be part and may be used in biochemical assays in kits for diagnostic purposes.

The present invention is further described by way of examples. Said examples illustrate the invention further without limiting the same thereto.

### Examples

### Methods

**Cells and reagents.** CHO-K1 (ACC-110) cells were from Leibniz Institute DSMZ-German Collection of Microorganisms and Cell Cultures (Braunschweig, Germany). U4C cells were kindly provided by Heike Hermanns (University Würzburg, Germany). Murine Ba/F3-gp130 cells transduced with human gp130 were provided by Immunex (Seattle, WA, USA). The packaging cell line Phoenix-Eco was obtained from Ursula Klingmüller (DKFZ, Heidelberg, Germany) (Ketteler, R., et al., Gene therapy 9, 477-487, 2002). Ba/F3-gp130 cells with murine IL-12Rβ1 and murine IL-23R have been described previously (Floss, D. M. et al. J Biol Chem 288, 19386-19400, 2013). All cell lines were grown in DMEM high glucose culture medium (GIBCO®, Life Technologies, Darmstadt, Germany) supplemented with 10% fetal calf serum (GIBCO®, Life Technologies), 60 mg/l penicillin and 100 mg/l streptomycin (Genaxxon Bioscience GmbH, Ulm, Germany) at 37°C with 5% CO₂ in a water saturated atmosphere. Ba/F3-gp130 cells were maintained in the presence of Hyper-IL-6 (HIL-6), a fusion protein of IL-6 and the soluble IL-6R, which mimics IL-6 trans-signaling. Either recombinant protein (10 ng/ml) or 0.2% (10 ng/ml) of conditioned cell culture medium from a stable CHO-K1 clone secreting Hyper-IL-6 (stock solution approx. 5 µg/ml as determined by ELISA) were used to supplement the growth medium. Ba/F3-IL-12Rβ1-IL-23R cells expressing murine IL-23R and murine IL-12Rβ1 were stimulated with 0.2% (10 ng/ml) of conditioned cell culture medium from a stable CHO-K1 clone secreting murine Hyper-IL-23 (HIL-23) in a concentration of approx. 5 µg/ml, as determined by ELISA. Phospho-STAT3 (Tyr705) (D3A7) (cat. #9145), STAT3 (124H6) (cat. #9139), phospho-p44/42 MAPK (ERK1/2) (Thr-202/Tyr-204) (D13.14.4E) (cat. #4370), p44/42 MAPK (ERK1/2) (cat. #9102), phospho-AKT (Ser473) (D9E) (cat. #4060), AKT (cat. #9272S), phospho-JAK1 (Tyr1022/1023) (cat. #3331), JAK1 (6G4) (cat. #3344S), phospho-JAK2 (Tyr1007/1008) (cat. #3771), JAK2 (D2E12) (cat. #3230), phospho-TYK2 (Tyr1054/1055) (cat. #9321), TYK2 (cat. #9312), GFP (4B10) (cat. #2955) and myc (71D10), (cat. #2278) monoclonal antibodies (mAbs) were obtained from Cell Signaling Technology (Frankfurt, Germany). mCherry (cat. 31451) was obtained from Thermo Fisher Scientific (Waltham, MA, USA). Flag (DYKDDDDK) (cat. F7425) and γ-tubulin (cat. T5326) mAbs were obtained from Sigma-Aldrich (Munich, Germany). Human CIS3/SOCS3 (C204) mAb (cat. JP18391) was obtained from Immuno-Biological Laboratories Co, Ltd. (Fujioka, Japan). β-actin (C4) mAb (cat. sc-47778) was obtained from Santa Cruz Biotechnology (Dallas, USA). Peroxidase-conjugated secondary mAbs (cat. 31462, cat. 31451) were obtained from Pierce (Thermo Fisher Scientific, Waltham, MA, USA). Alexa Fluor 488 conjugated Fab goat anti-rabbit IgG (cat. A11070) was obtained from Thermo Fisher Scientific (Waltham, MA, USA)

### Construction of Synthetic Cytokine Receptors (SyCyRs) and synthetic ligands.

pcDNA3.1-G_{VHH}-IL-23R expression vector was generated by fusion of coding sequences for human IL-11R signal peptide (Q14626, AS 1-24) followed by sequences for myc tag, GFP-nanobody (G_{VHH}) (Rothbauer, U. et al. Mol Cell Proteomics 7, 282-289, 2008) and murine IL-23R (Q8K4B4, Uniprot) comprising amino acids A358 to K644 representing 17 aa of the extracellular domain, the transmembrane domain and the cytoplasmic part of the receptor. The cDNA coding for G_{VHH}-IL-12Rβ1 was generated by insertion of cDNA coding for murine IL-12Rβ1 (Q60837, Uniprot, aa A551-A738, representing 15 aa of the extracellular domain, the transmembrane domain and the cytoplasmic part of the receptor), which was amplified by PCRfrom p409-IL-12Rβ1, see Foss et al., into expression vector pcDNA3.1-G_{VHH}-IL-23R, where the coding sequence for IL-23R was removed. pcDNA3.1-C_{VHH}-IL-23R expression vector was generated by fusion of coding sequences for human IL-11R signal peptide (Q14626, AS 1-24) followed by sequences for Flag-tag, mCherry-nanobody (C_{VHH}) (Fedorov, V. D., et.al., Sci Transl Med 5, 215ra172, doi:10.1126/scitranslmed.3006597, 2013) and murine IL-23R (Q8K4B4) comprising amino acids A358 to K644 (representing 17 aa of the extracellular domain, the transmembrane domain and the cytoplasmic part of the receptor). To combine cDNAs coding for C_{VHH}-IL-23R and G_{VHH}-IL-12Rβ1 in one open reading frame, cDNAs coding for both SyCyRs were amplified by PCR and cloned into pMK-FUSIO coding for the self-processing 2A-peptide (Fang, J. et al. Nat Biotechnol 23, 584-590, 2005). The cDNA coding for IL-23R-ΔSTAT (Δ503) was amplified by PCR from pcDNA3.1-IL-23R-ΔSTAT (Δ503), see Foss above, and inserted into pcDNA3.1-G_{VHH}-IL-23R, where the sequence for IL-23R was removed. Accordingly, cDNAs coding for ΔJAK-A (IL-23R-Δ403-417), ΔJAK-B (IL-23R-Δ455-479) and ΔJAK-C (IL-23R-Δ403-479) were amplified by PCR from p409-IL-23R-Δ403-417, -IL-23R-Δ455-479 and -IL-23R-Δ403-479 (Floss, D. et al. Mol Biol Cell 27, 2301-2316, doi:10.1091/mbc.E14-12-1645 2016) containing 16 aa of the extracellular domain, the transmembrane domain and the shortened cytoplasmic part of the receptor and inserted into pcDNA3.1-C_{VHH}-IL-23R, where the coding sequence for IL-23R was removed. p409-gp130 (chemically synthesized by GeneArt, Thermo Fisher Scientific, Waltham, MA, USA) was digested by EcoRI, NotI and ligated in pcDNA3.1-G_{VHH}-IL-23R, which was digested by the same enzymes to generate pcDNA3.1-G_{VHH}-gp130 containing 16 aa of the extracellular domain, the transmembrane domain and the intracellular domain of the human receptor. cDNA coding for gp130-ΔSTAT was amplified by PCR (aa 1-758) and ligated to the same vector mentioned before. The expression cassette for 3xGFP was obtained from pmEGFP-13 (Addgene, Cambridge, MA, USA) and inserted into pcDNA3.1 expression vector containing the IL-11 R signal peptide and an N-terminal Flag-tag. To generate pcDNA3.1-2xGFP-mCherry, one GFP from pcDNA3.1-3xGFP was removed and replaced with mCherry from pcDNA3.1-mCherry. To create single GFP, the cDNA coding for GFP was amplified by PCR from pcDNA3.1-2xGFP-mCherry and inserted into the pcDNA3.1 expression vector. To create pcDNA3.1-GFP-mCherry, cDNA coding for mCherry was inserted into pcDNA3.1-GFP. For retroviral transduction of Ba/F3-gp130 cells two retroviral plasmids with different resistance genes, pMOWS-puro coding for puromycin resistance and pMOWS-hygro for hygromycin B resistance, have been used. Expression cassettes coding for C_{VHH}-IL-23R-2A-G_{VHH}-IL-12Rβ1 (SyCyR(IL-23/2A)), C_{VHH}-IL-23R, C_{VHH}-IL-23R-ΔJAK-A (Δ403-417), C_{VHH}-IL-23R-ΔJAK-B (Δ455-479), C_{VHH}-IL-23R-ΔJAK-C (Δ403-479), and G_{VHH}-gp130 were inserted into pMOWS-puro, whereas those for G_{VHH}-IL-12Rβ1, G_{VHH}-IL-23R-ΔSTAT (Δ503) and G_{VHH}-gp130-ΔSTAT were inserted into pMOWS-hygro. All generated expression plasmids have been verified by sequencing.

**Transfection, transduction and selection of cells.** Transfection of U4C and CHO-K1 cells with indicated plasmids was performed using TurboFect™ (Thermo Fisher Scientific, Waltham, MA, USA). Ba/F3-gp130 cells were retrovirally transduced with the pMOWS expression plasmids coding for the various synthetic receptor variants (see Floss et al.). Transduced cells were grown in standard DMEM medium as described above supplemented with 10 ng/ml HIL-6. Selection of transduced Ba/F3 cells was performed with puromycin (1.5 µg/ml) or hygromycin B (1 mg/ml) (Carl Roth, Karlsruhe, Germany) or both for at least two weeks. Afterwards, HIL-6 was washed away and the generated Ba/F3-gp130 cell lines were selected for GFP:mCherry-dependent growth and analyzed for receptor cell surface expression. Stable transfected CHO-K1 cells secreting GFP:mCherry proteins were selected with 1.125 mg/ml G-418 sulfate (Genaxxon, Biosciences, Ulm, Germany). High expressing cell clones were identified by Western Blotting.

**Expression and purification of G_{VHH}-C_{VHH} from *E.coli.*** The cDNA coding for the bispecific antibody G_{VHH}-C_{VHH} was generated and subcloned in pet23a. The resulting bispecific antibody sequence was flanked by an N-terminal PeIB leader sequence for periplasmic expression and a 3' hexahistidine sequence for purification. Proteins were expressed in the *E.coli* strain BL21-Rosetta. Bacteria were incubated in two liters LB-media containing ampicillin 1:1000 (100 µg/ml) and chloramphenicol 1:1000 (34 µg/ml) at 37°C, until optical density reached 0.6-0.9. Then 1 mM IPTG was added. Bacteria were harvested by centrifugation (5000 x g, 30 min, 4°C) 4 hours after IPTG induction. A cOmplete protease inhibitor tablet (Roche, Mannheim, Germany) was added and supernatant was filtered through a 0.45 µm bottle top filter. Proteins were purified via IMAC chromatography and eluted with 500 mM imidazole.

**Cell viability assay.** To remove the cytokines, Ba/F3-gp130 cell lines were washed 3 times with sterile PBS. 5 x 10³ cells were suspended in DMEM supplemented with 10% FCS, 60 mg/l penicillin and 100 mg/l streptomycin and cultured for three days in a final volume of 100 µl with or without cytokines/fluorescent proteins as indicated. The CellTiter-Blue Cell Viability Assay (Promega, Karlsruhe, Germany) was used to estimate the number of viable cells by recording the fluorescence (excitation 560 nm, emission 590 nm) using the Infinite M200 PRO plate reader (Tecan, Crailsheim, Germany) immediately after adding 20 µl of reagent per well (time point 0) and up to 2 h after incubation under standard cell culture conditions. All of the values were measured in triplicate per experiment. Fluorescence values were normalized by subtraction of time point 0 values.

**Stimulation assays.** For analysis of STAT3, ERK1/2 and AKT, JAK1, JAK2 and TYK2 activation Ba/F3-gp130 cell lines expressing various SyCyR variants were washed three times with sterile PBS and incubated in serum-free DMEM for at least 2 h. Cells were stimulated with GFP:mCherry fusion proteins as indicated, harvested, frozen in liquid nitrogen and lysed. Protein concentration of cell lysates was determined by BCA Protein Assay (Pierce, Thermo Fisher Scientific,Waltham, MA, USA). Analysis of STAT3, ERK1/2, AKT, JAK1, JAK2 and TYK2 activation, SOCS3 and β-actin expression was done by immunoblotting using 25-75 µg proteins from total cell lysates and detection with phospho-STAT3, phospho-ERK1/2, phospho-AKT, phospho-JAK1, phospho-JAK2, phospho-TYK2, SOCS3 and β-actin mAbs.

**Western blotting.** Defined amounts of proteins from cell lysates were loaded per lane, separated by SDS-PAGE under reducing conditions and transferred to PVDF membranes (Carl Roth, Karlsruhe, Germany). The membranes were blocked in 5% fat-free dried skimmed milk (Carl Roth, Karlsruhe, Germany) in TBS-T (10 mM Tris-HCl (Carl Roth, Karlsruhe, Germany) pH 7.6, 150 mM NaCl (AppliChem, Darmstadt, Germany), 1% Tween 20 (Sigma Aldrich, Munich, Germany)) and probed with the indicated primary antibodies in 5% fat-free dried skimmed milk in TBS-T (STAT3, β-actin, GFP mAbs) or 5% BSA (Carl Roth, Karlsruhe, Germany) in TBS-T (phospho-STAT3, phospho-ERK1/2, ERK1/2, phospho-AKT, AKT, myc and Flag, SOCS3, phospho-JAK1, JAK1, phospho-JAK2, JAK2, phospho-TYK2, TYK2 and β-actin mAbs) at 4°C overnight. After washing, the membranes were incubated with secondary peroxidase-conjugated antibodies (Thermo Fisher Scientific, Waltham, MA, USA) diluted in 5% fat-free dried skimmed milk in TBS-T for 1 h at room temperature. The Immobilon™ Western Chemiluminescent HRP Substrate (Merck Chemicals GmbH, Darmstadt, Germany) and the ChemoCam Imager (INTAS Science Imaging Instruments GmbH, Göttingen, Germany) were used for signal detection. For re-probing with another primary antibody, the membranes were stripped in 62.5 mM Tris-HCl (Carl Roth, Karlsruhe, Germany) pH 6.8, 2% SDS (Carl Roth, Karlsruhe, Germany) and 0.1% β-mercaptoethanol (Sigma Aldrich, Munich, Germany) for 30 min at 60°C and blocked again.

**Cell surface detection of cytokine receptors.** To detect cell surface expression of the synthetic cytokine receptors, stably transduced Ba/F3-gp130 cells were washed with FACS buffer (PBS containing 1 % BSA) and incubated at 5 x 10⁵ cells/100 µl FACS buffer supplemented with a 1:100 dilution of anti-myc or 1.2 µg anti-Flag mAbs for 1 h on ice. After a single wash with FACS buffer, cells were incubated in 100 µl FACS buffer containing a 1:100 dilution of Alexa Fluor 488 conjugated Fab goat anti-rabbit IgG for 1 h on ice. Finally, cells were washed once with FACS buffer, suspended in 500 µl FACS buffer and analyzed by flow cytometry (BD FACSCanto II flow cytometer, BD Biosciences, San Jose, CA, USA). Data was evaluated using the FCS Express 4 Flow software (De Novo Software, Los Angeles, CA, USA).

**Microarray analysis.** Ba/F3-gp130 cells were grown in DMEM high glucose culture medium supplemented with 10% fetal calf serum (GIBCO®, Life Technologies), 60 mg/l penicillin and 100 mg/l streptomycin (Genaxxon Bioscience GmbH, Ulm, Germany) at 37°C with 5% CO₂ in a water saturated atmosphere. 10 ng/ml of conditioned cell culture medium from a stable CHO-K1 clone secreting Hyper-IL-6 (stock solution approx. 5 µg/ml as determined by ELISA) were used to supplement the growth medium. Ba/F3-gp130 cells were stable transduced with different receptor complexes (IL-12Rβ1-IL-23R, C_{VHH}-IL-23R-2A-G_{VHH}-IL-12Rβ1(SyCyR(IL-23/2A)) or G_{VHH}-IL-23R (SyCyR(IL-23R))), independently selected and cultivated for several weeks. Subsequently, Ba/F3-gp130 cell lines were washed four times with sterile PBS and incubated in serum-free DMEM for 3 h. Equal numbers of cells (2 x 10⁶) were stimulated with 100 ng/ml HIL-23, GFP-mCherry or 3xGFP for 1 h at 37°C, independently. Stimulation with cell culture supernatant from untransfected CHO-K1 cells was used as control. Total RNA extraction of four independent biological replicates was made with RNeasy Mini Kit (Qiagen, Hilden, Germany) according to the manufacturer's instructions. RNA quality was evaluated using an Agilent 2100 Bioanalyzer and only high-quality RNA (RIN > 8) was used for microarray analysis. For this total RNA (150 ng) was processed using the Ambion WT Expression Kit and the WT Terminal Labeling Kit (Thermo Fisher Scientific, Waltham, MA, USA) and hybridized on Affymetrix Mouse Gene ST 1.0 arrays containing about 28,000 probe sets. Staining and scanning were done according to the Affymetrix expression protocol. Expression console (Affymetrix, Freiburg, Germany) was used for quality control and to obtain annotated normalized RMA gene-level data (standard settings including sketch-quantile normalization). Statistical analyses were performed by utilizing the statistical programming environment R (R Development Core Team) implemented in CARMAweb (1.5-fold, p-value ≤ 0.01). Data were analyzed pair-wise, Ba/F3-SyCyR(IL-23/2A) cells stimulated with 100 ng/ml GFP-mCherry versus Ba/F3-IL-12Rβ1-IL-23R cells stimulated with 100 ng/ml HIL-23 and Ba/F3-SyCyR(IL-23R) cells stimulated with 100 ng/ml 3xGFP versus Ba/F3-SyCyR(IL-23/2A) cells stimulated with 100 ng/ml GFP-mCherry.

GO term and pathway enrichment analyses (p < 0.01 of enrichment) of differential abundant transcripts (1.5-fold, p-value ≤ 0.01) were done with Ingenuity software (Qiagen, Hilden, Germany). Gene expression raw data are available at GEO (accession number GSE101569).

**Animals.** C57BL/6 mice (Janvier Labs) were obtained from the animal facility of the University of Düsseldorf. Mice were fed with a standard laboratory diet and given autoclaved tap water ad libitum. They were kept in an air-conditioned room with controlled temperature (20-24°C), humidity (45-65%), and day/night cycle (12 h light, 12 h dark). Mice were acclimatized for 1 week before entering the study. All procedures were performed in accordance with the national guidelines for animal care and were approved by the local Research Board for animal experimentation (LANUV, State Agency for Nature, Environment and Consumer Protection, approval number (Az. 84-02.04.2016.A025)).

**Hydrodynamic-based *in vivo* gene delivery.** 8 week-old male C57BL/6 mice were transfected via tail vein injection of the plasmids pcDNA3.1-G_{VHH}-gp130 (2.3 µg/mouse) and/or pcDNA3.1-3xGFP (1.28 µg/mouse) prepared in PBS as described previously.

**Preparation of liver lysates.** Tissue protein extracts from liver were prepared on ice using the lysis buffer (50 mM Tris-HCl (Carl Roth, Karlsruhe, Germany), 150 mM NaCl (AppliChem, Darmstadt, Germany), 2 mM EDTA (Sigma Aldrich, Munich, Germany), 2 mM NaF (Sigma Aldrich, Munich, Germany), 1 mM Na₃VO₄ (Sigma Aldrich, Munich, Germany), 1% Nonidet P40 BioChemica (AppliChem, Darmstadt, Germany) 1% Triton X-100 (Sigma Aldrich, Munich, Germany) and cOmplete EDTA-free Protease inhibitor cocktail tablet (Roche Diagnostics, Mannheim, Germany) and analyzed by Western blotting. Equal amounts of protein (50 µg/lane) were loaded.

**Statistical analysis.** Data are presented as mean ± SEM. For multiple comparisons, one-way ANOVA, followed by Bonferroni post hoc tests, was used (GraphPad Prism 6.0, GraphPad Software Inc., San Diego, CA, USA). Statistical significance was set at the level of *p* < 0.05.

### RESULTS

### Synthetic Cytokine Receptors (SyCyRs) simulate IL-23 and IL-6/IL-11 signaling.

Natural biological switches regulate cytokine-induced signal transduction via receptor activation and inactivation. Highly specific nanobodies against GFP and mCherry were selected as tools to mediate sensitive and background free cytokine-like signaling. Naturally, IL-23 signals via its receptor complex consisting of IL-23R and IL-12Rβ1. To mimic IL-23 signaling, we generated two Synthetic Cytokine Receptors (SyCyRs) in which the extracellular part, including the ligand-binding site of the IL-23R and the IL-12Rβ1 was replaced by nanobodies specifically recognizing mCherry (C_{VHH}) and GFP (G_{VHH}), respectively (Fig. 1a, Fig. 1c). SyCyRs were expressed in Ba/F3-gp130 cells, which proliferate following STAT3 activation by the fusion protein of IL-6 and soluble IL-6R named Hyper-IL-6 (HIL-6) (Fig. 1 b). As synthetic ligands for SyCyRs, we generated GFP-mCherry fusion proteins and various combinations (Fig. 1a, Fig. 1). Since STAT3 activation is a hallmark of IL-23 signal transduction, we tested if STAT3-dependent proliferation of Ba/F3-gp130 cells expressing C_{VHH}-IL-23R and G_{VHH}-IL-12Rβ1 (Ba/F3-SyCyR(IL-23/2A) generated using 2A-technology with both cDNAs combined in one open reading frame and Ba/F3-SyCyR(IL-23) generated with two separate cDNAs) could be induced with GFP and mCherry proteins and fusions of these. Interestingly, proliferation of Ba/F3-SyCyR(IL-23/2A) cells was specifically induced by GFP-mCherry and 2xGFP-mCherry fusion proteins, but not by single GFP and mCherry proteins (Fig. 1 b). Ba/F3 cells only expressing C_{VHH}-IL-23R or G_{VHH}-IL-12Rβ1 failed to proliferate, demonstrating the high selectivity of GFP-mCherry as a synthetic cytokine ligand. Comparative analysis of the dose-dependent proliferation of Ba/F3-SyCyR(IL-23/2A) and Ba/F3-IL-12Rβ1-IL-23R cells with GFP-mCherry and Hyper-IL-23 (HIL-23), respectively, revealed that the natural cytokine and synthetic cytokine exhibited comparable potency with half-maximal proliferation achieved with HIL-23 (56 kDa) and GFP-mCherry (57 kDa) concentrations of about 5-10 ng/ml. GFP-mCherry-induced cellular proliferation of Ba/F3-SyCyR(IL-23/2A) cells was specifically inhibited by a soluble G_{VHH}-C_{VHH} fusion protein (Fig. 1e). Analysis of intracellular signal transduction showed that the GFP-mCherry, 2xGFP-mCherry fusion proteins, but not single mCherry, GFP and 3xGFP proteins induced JAK, STAT3, ERK1/2 and AKT phosphorylation in Ba/F3-SyCyR(IL-23/2A) cells. SyCyR(IL-23/2A) also resulted in specific STAT3 activation in U4C cells. Kinetics of pSTAT3 and SOCS3 induction following of Ba/F3-SyCyR(IL-23/2A) and Ba/F3-IL-12Rβ1-IL-23R cells with either GFP-mCherry or HIL-23 were comparable. Since the IL-23R complex is not targeted by SOCS3, activation resulted in sustained STAT3 phosphorylation. Next, we analyzed the mRNA-expression by gene-array analysis of Ba/F3-SyCyR(IL-23/2A) cells stimulated with GFP-mCherry and Ba/F3-IL-12Rβ1-IL-23R cells stimulated with HIL-23. GFP-mCherry and HIL-23 up- or down-regulated 107 and 193 genes, respectively, by a factor 1.5 or more. Among them are typical STAT3-target genes, including PIM1, SOCS3 and OSM. Pathway analysis revealed that the genes regulated by GFP-mCherry and HIL-23 belong to the same pathways. Our data revealed a high degree of overlap between the transcriptome induced by the synthetic ligand as compared to the natural cytokine.

To investigate whether SyCyRs can be activated by homodimeric ligands, we adapted this system to the IL-6/IL-11 receptor complex. A SyCyR for gp130 was generated in which the extracellular part of the cytokine receptor was replaced by G_{VHH} (G_{VHH}-gp130), (Fig. 2a, Fig. 1c). Expression of G_{VHH}-gp130 in Ba/F3-gp130 cells (Ba/F3-SyCyR(IL-6)) was verified by flow cytometry. As expected, JAK, TYK, STAT3, ERK1/2 phosphorylation in Ba/F3-SyCyR(IL-6) cells was specifically induced by 2xGFP-mCherry and 3xGFP. Comparison of the dose-dependent proliferation of Ba/F3-SyCyR(IL-6) and Ba/F3-gp130 with 3xGFP and HIL-6, respectively, showed that the natural and synthetic cytokine exhibited similar activity with half-maximal proliferation at 1-10 ng/ml 3xGFP (86 kDa) or HIL-6 (60 kDa), respectively. 3xGFP-stimulation of Ba/F3-SyCyR(IL-6) cells resulted in time-dependent fast activation and slight inactivation of STAT3 phosphorylation after 120 min which was accompanied by up-regulation of SOCS3. Overall, 3xGFP-induced signal transduction was undistinguishable from HIL-6 induced STAT3-phosphorylation and SOCS3 expression. Next, we expressed SyCyR(IL-6) in liver tissue of C57BL/6 mice. 24 h after injection of cDNAs coding for G_{VHH}-gp130 and 3xGFP alone or in combination we observed STAT3 phosphorylation when G_{VHH}-gp130 was coexpressed with 3xGFP (Fig. 2b). Interestingly, G_{VHH}-gp130 expression was found to be much higher in mice injected only with the cDNA coding for G_{VHH}-gp130 as compared to mice expressing both, G_{VHH}-gp130 and 3xGFP. The data suggest that co-expression of G_{VHH}-gp130 and 3xGFP resulted in activation-dependent degradation of G_{VHH}-gp130. Moreover, detection of 3xGFP in serum samples by Western blotting showed strong accumulation of 3xGFP in mice injected only with the cDNA coding for 3xGFP, whereas 3xGFP was hardly detectable in mice injected with cDNAs coding for G_{VHH}-gp130 and 3xGFP. These findings suggest that not only G_{VHH}-gp130 but also 3xGFP protein was efficiently internalized and degraded in liver cells after binding and activation of G_{VHH}-gp130. Consistently, expression of the acute phase response gene *Saa1* was increased following injection of cDNAs coding for G_{VHH}-gp130 and 3xGFP, in sharp contrast to injection of cDNA coding for G_{VHH}-gp130 or 3xGFP alone. Overall, our data showed that the tested SyCyRs phenocopied IL-6 and IL-23 signaling *in vitro* and *in vivo.*

### Synthetic IL-23R cytokine receptor homodimers are biologically active.

Since multimeric GFP was able to induce IL-6 signal transduction via homodimeric G_{VHH}-gp130, we wondered, whether IL-23R is also biologically active as a homodimer. Accordingly, we created a SyCyR consisting of the extracellular G_{VHH} fused to the transmembrane and intracellular domains of the IL-23R (G_{VHH}-IL-23R; (SyCyR(IL-23R)) (Fig. 3a and Fig. 1c). Interestingly, 3xGFP and 2xGFP-mCherry fusion proteins induced proliferation of Ba/F3-SyCyR(IL-23R) cells (Fig. 3b), whereas single GFP and mCherry or the heterodimeric GFP-mCherry fusion protein did not (Fig. 3b). Half-maximal proliferation was achieved at about 10-20 ng/ml 3xGFP (86 kDa), which was only slightly lower as compared to Ba/F3-IL-12Rβ1-IL-23R cells stimulated with HIL-23 (56 kDa; 5-10 ng/ml). Thus the slightly reduced activity cannot be explained through differences in the molar concentrations of the molecules. As expected, 3xGFP and 2xGFP-mCherry fusion proteins but not single GFP induced JAK, STAT3, pERK1/2 and AKT phosphorylation in Ba/F3-SyCyR(IL-23R) cells. Also the kinetics of pSTAT3 activation and SOCS3 expression of HIL-23 and 3xGFP were comparable, suggesting that also IL-23R homodimers were not negatively regulated by SOCS3. Surprisingly, only 35 genes were up- or down-regulated by at least 1.5-fold after stimulation of Ba/F3-SyCyR(IL-23R) with 3xGFP. However, 34 out of 35 transcripts were also found in the GFP-mCherry-group (Ba/F3-SyCyR(IL-23/2A)). Among the 35 regulated genes are typical IL-23 target genes, including PIM, SOCS3, and OSM. Although, a reduced number of genes was triggered by homodimeric IL-23R signaling when compared to IL-12Rβ1-IL-23R heterodimer stimulation, similar signaling pathways were affected. In conclusion, homotypic activation of SyCyR(IL-23R) also phenocopied IL-23 signaling in terms of signal transduction pathways and kinetics, but resulted in overall reduced induction of gene expression as compared to SyCyR(IL-23/2A).

### Engineered heterotrimeric SyCyRs are capable of STAT3 trans-phosphorylation.

During trans-phosphorylation a kinase-active receptor is able to trans-phosphorylate a kinase-negative mutant receptor. Since 2xGFP-mCherry was able to induce functional hetero- and homodimerization of SyCyRs, we wondered whether 2xGFP-mCherry can induce trans-phosphorylation of STAT3 via synthetic trimeric receptor complexes. Hence, a C-terminally truncated IL-23R (Δ503), lacking the canonical STAT binding motifs but retained JAK, ERK and AKT activity (IL-23R-ΔSTAT) was selected and fused with G_{VHH} (Fig. 4a and 4b). Janus kinases interact with peptide motifs within the IL-23R localized between amino acid 403-479 and complete or partial deletion results in disabled JAK activity. Accordingly, we created three deletion variants of the IL-23R intracellular domain with disabled JAK activity, ΔJAK-A (Δ403-417), ΔJAK-B (Δ455-479) and ΔJAK-C (Δ403-479) fused to the mCherry-nanobody (Fig. 4a and Fig. 4b). Cell surface expression of these SyCyRs in Ba/F3-gp130 cells was verified by flow cytometry. As expected, stimulation of G_{VHH}-IL-23R-ΔSTAT in Ba/F3-gp130 cells with 2xGFP-mCherry resulted in JAK and ERK phosphorylation but defective STAT3 activation (Fig. 4c). Consequently, 3xGFP-induced proliferation of Ba/F3-G_{VHH}-IL-23R-ΔSTAT cells was drastically reduced as compared to Ba/F3-SyCyR(IL-23R) cells (Fig. 4d). Interestingly, only the assembly of a 2xGFP-mCherry-induced trimeric complex consisting of two G_{VHH}-IL-23R-ΔSTAT receptors and one C_{VHH}-IL-23R-ΔJAK receptor resulted in increased STAT3 trans-phosphorylation and cellular proliferation (Fig. 4e). Dimerization of G_{VHH}-IL-23R-ΔSTAT with all C_{VHH}-ΔJAK receptors by GFP-mCherry did not induce STAT activation, demonstrating that two biologically active JAKs in G_{VHH}-IL-23R-ΔSTAT were needed for STAT3 trans-phosphorylation. Of note, also stimulation with a 2xmCherry fusion protein and formation of dimeric C_{VHH}-IL-23R-ΔJAK did not result in STAT3 phosphorylation, whereas homodimers of C_{VHH}-IL-23R were biologically active, demonstrating that the C_{VHH}-IL-23R-ΔJAK variants were not biologically active as dimers.

### Discussion

Here, we describe the development of a synthetic cytokine receptor system based on nanobodies directed against GFP and mCherry fused to truncated cytokine receptors. Nanobodies are versatile tools widely used in molecular biology, exhibiting high affinity and antigen specificity. We chose nanobodies against GFP and mCherry because these fluorescent proteins are non-toxic to mammalian cells, will not cause unspecific binding to endogenous receptors and are therefore considered as side-effect/background-free. As receptor system, we used heterodimeric and homodimeric cytokine receptor compositions exemplified by IL-23 and IL-6 receptor signaling complexes. IL-23 signals via a heterodimeric receptor complex consisting of IL-12Rβ1 and IL-23R, whereas IL-6 signals via the non-signal transducing IL-6R and the signal-transducing homodimer of gp130. Both receptor complexes induce signals via receptor-associated Janus kinases that activate STAT, ERK and AKT pathways. JAKs are constitutive but non-covalently associated with class I and II cytokine receptors, which upon cytokine binding bring together two JAKs to create an active signaling complex. JAK interact with receptor peptide motifs which are present in the intracellular domain of cytokine receptors. During receptor activation, JAKs switch into the "on"-status by reciprocal phosphorylation and subsequent phosphorylation of receptor-tyrosines and signaling molecules such as STAT3.

The synthetic receptor complex mimicking IL-23-signaling was activated by a heterodimeric synthetic GFP-mCherry ligand but not by single GFP or mCherry or multimeric GFP fusion proteins, whereas the synthetic receptor complex simulating IL-6-signaling was specifically activated by homodimeric synthetic GFP-ligands. Importantly, GFP-mCherry and 3xGFP fusion proteins did not activate cellular responses in cells lacking synthetic cytokine receptors.

A recent report showed that not only the intracellular domains determine the signaling strength but also the mode of extracellular receptor complex assembly. Specifically, a point mutation in EPO was shown to change EPO receptor dimerization, which resulted in reduced STAT1 and STAT3 phosphorylation but did not affect STAT5 activation. This implies, that replacing the extracellular part of a cytokine receptor by another binding domain, such as nanobodies might influence signaling strength and kinetics, which ultimately lead to an altered intracellular response of the chimeric receptor. To exclude such effects for our synthetic cytokine receptors, apart from general analysis of typical signal transduction pathways (JAK/STAT, ERK, AKT), we verified that the time-dependent activation profile of IL-23 and IL-6 is identical with those of synthetic ligands. These findings were supported by transcriptome comparison of Ba/F3-IL-23R-IL-12Rβ1 and Ba/F3-SyCyR(IL-23/2A) cells, activated by HIL-23 and GFP-mCherry, respectively, in which almost all regulated genes for both cytokines were identical. Even though more regulated genes were detected after HIL-23 stimulation as compared to GFP-mCherry stimulation, this difference was within expected fluctuations when using cell lines, which have been transduced with different receptor complexes and have been independently selected and cultivated for several weeks. Importantly, pathway analysis of the natural and synthetic IL-23 receptor complexes highlighted that naturally and synthetically induced signal transduction was basically identical. This is the first study using chimeric receptor complexes, which included a detailed analysis of signal transduction pathways and expression profiles. Importantly, the synthetic receptors appear to be active *in vivo,* since we demonstrated the activation of G_{VHH}-gp130 by 3xGFP in the liver of mice after hydrodynamic injection.

So far, no signaling role of IL-12Rβ1 in the receptor complex apart from activation of a Janus kinase has been assigned. Consistently, using our synthetic receptors, we induced IL-23R homodimeric receptor complexes. Although the general activation of signaling pathways appeared to be similar to IL-23 signaling, gene-array analysis revealed a reduced number of regulated genes when compared to heterodimeric signaling. This phenomenon could be caused by the slightly reduced proliferation observed following SyCyR(IL-23R) signaling when compared to natural IL-23R-IL-12Rβ1 complex. Moreover, reduced affinity or biochemical features of the synthetic ligand may affect SyCyR signaling. This effect might also contribute to the observation, that the natural and synthetic IL-23 receptor activation was different in terms of the absolute number of regulated genes.

The modular nature of the synthetic ligands, with one receptor binding site per GFP or mCherry allows an exact composition of the receptor stoichiometry, which clearly will be interesting for many if not all other cytokine receptors. Moreover, this system will enable the combinatory assembly of novel receptor combinations with desirable signaling potentials and capacities. The number of recruited synthetic receptors is only limited by the maximal number of ligands connected in one GFP:mCherry fusion protein or by alternative GFP/mCherry multimerization strategies.

For IL-6 and IL-23 signaling, we used homo- and heterodimeric GFP:mCherry fusion proteins, but we were also able to generate homo- and heterotrimeric GFP:mCherry variants. Using these synthetic ligands, we analyzed, if biologically active trimeric receptor complexes could also be functionally assembled among the cytokine receptor family with associated kinases. Tyrosine-receptors and receptors with associated kinases are typically active as dimers to juxtapose and subsequently activate at least two receptor kinases. This implies that these receptor systems naturally did not require a third receptor. To generate trimeric receptor complexes for IL-6 and IL-23-simulations, we deleted the STAT3-binding motifs in the synthetic G_{VHH}-IL-23 and G_{VHH}-gp130 receptors and combined these receptors with JAK-deficient receptors containing STAT-binding motifs fused to C_{VHH}. We showed that in these trimeric receptor combinations, STAT3 activation is mediated by trans-phosphorylation. The formation of a trimeric receptor complex with two JAK-proficient but STAT-deficient receptors and one STAT-binding motif receptor by GFP-GFP-mCherry (2xGFP-mCherry) resulted in STAT3 trans-phosphorylation. Assembly of one JAK-proficient receptor with one STAT-binding motif receptor by a GFP-mCherry fusion did, however, not lead to trans-phosphorylation, confirming that one Janus kinase is not sufficient for receptor activation. Of note, trans-phosphorylation was thus far only described for tyrosine-kinase-receptors of the PDGF and EGF family, in which a kinase-active receptor was able to trans-phosphorylate a second kinase-inactive mutant receptor after receptor dimerization. In these cases, the kinase-negative mutant receptor was able to activate the functional kinase of the other receptor. Here, we describe for the first receptor-chain trans-phosphorylation for cytokine receptors with associated Janus kinases.

In summary, the synthetic cytokine receptor system allows tailor-made activation and analysis of cytokine signaling by recruitment of defined numbers and compositions of receptor chains. Receptor assembly is determined by the number and sequence of GFP-mCherry units in the ligand fusion proteins. This system simulates signal transduction without relevant back-ground activation that has been described previously with chimeric receptor systems. The lack of toxicity of fluorescent proteins *in vitro* and *in vivo* allows a widespread area of potential applications for studying cell-type specific receptor activation by synthetic ligand application in transgenic mice. Importantly, our system is easily on/off-switchable, because signal activation can be rapidly inhibited by application of soluble nanobody-fusion proteins directed against the synthetic GFP:mCherry ligands and will open up novel therapeutic regimes involving non-physiological targets during immunotherapy.

## Claims

1. A recombinant polypeptide containing at least the following domains starting from the N-terminus to the C-terminus:
a first domain containing a first binding partner selected from an artificial ligand and a receptor binding an artificial ligand;
optionally a spacer domain,
a transmembrane domain, and
a cytoplasmic signaling domain wherein the first domain and the cytoplasmic signaling domain is a combination of domains not naturally occurring in an organism.

2. The recombinant polypeptide according to claim 1 wherein the first binding partner is selected from the group consisting of a single chain antibody unit, a receptor molecule, an endogenous peptide or a fragment thereof, and an artificial peptide.

3. The recombinant polypeptide according to claim 1 or claim 2 further comprising a leader sequence being located N-terminally to the first domain containing a first binding partner, like a single chain antibody unit.

4. The recombinant polypeptide according to any one of the preceding claims wherein the cytoplasmic signaling domain is derived from a cytokine receptor in particular is a cytoplasmic signaling domain selected from the group consisting of generally stimulating receptors, e.g. gp130, IL-23R, IL-12Rβ1, IL-7R, IL-13R, IL-15R, IFNαR, IFNγR, TNF1R, TNF2R, EGF-R, IL-22R, silencing (exhausting) receptors, e.g. PD-1, IL-10R, TIM3, IL-27R or killing receptors, e.g. FasR, TRAILR or is derived from a B-cell receptor or T-cell receptor, in particular, a cytoplasmic signaling domain selected from the group consisting of CD28, CD3 zeta chain, CD3 epsilon chain.

5. The recombinant polypeptide according to any one of the preceding claims wherein the transmembrane domain is a transmembrane domain derived from the cytokine receptor the cytoplasmic signaling domain is derived from.

6. The recombinant polypeptide according to any one of the preceding claims wherein the first domain containing the first binding partner is a first domain containing a nanobody.

7. A nucleic acid molecule comprising a nucleic acid sequence encoding the polypeptide according to any one of claims 1 to 6.

8. A vector comprising the nucleic acid sequence according to claim 7, in particular, a viral vector or a plasmid.

9. A cell, cell line or host cell containing a vector according to claim 8 or a nucleic acid molecule according to claim 7.

10. The cell, cell line or host cell according to claim 9 being modified peripheral blood cells, in particular, being lymphocytes including T-cells, like CD8+ or CD4+ T-cells, e.g. being a genetically engineered T-cell further expressing a chimeric antigen receptor.

11. The cell, cell line or host cell according to any one of claims 9 or 10 containing further a second vector according to claim 8 or a second nucleic acid molecule according to claim 7 wherein this vector or nucleic acid molecule encode a polypeptide according to any one of claims 1 to 6 being different in at least one of the first binding domain or the cytoplasmic signaling domain or both.

12. The cell, cell line or host cell according to claim 11 wherein the cytoplasmic signaling domain of the second vector induce signaling through different signaling pathways.

13. The recombinant polypeptide according to any one of claims 1 to 6, the nucleic acid molecule according to claim 7, the vector according to claim 8 or the cell, cell line or host cell according to any one of claims 9 to 12 for use in treating cancer or autoimmune disease or immune modulation of a subject.

14. The recombinant polypeptide according to any one of claims 1 to 6, the nucleic acid molecule according to claim 7, the vector according to claim 8 or the cell, cell line or host cell according to any one of claims 9 to 12 for use to change the status of cells when applying the specific second binding partner of the first binding partner present in said polypeptide, vector, nucleic acid molecule, or host cell, cell or cell line, in particular for use according to claim 13.

15. A kit or system containing a vector according to claim 8, a cell, cell line or host cell according to any one of claims 9 to 12, the nucleic acid molecule according to claim 7 and/or the peptide according to any one of claims 1 to 6 for use in the production of cells, in particular, lymphocytes, like T-cells, for immune modulation.
